(19) Europäisches Patentamt
European Patent Office
Office européen des brevets

(11) Veröffentlichungsnummer: **0 341 476**
**A2**

(12) # EUROPÄISCHE PATENTANMELDUNG

(21) Anmeldenummer: **89107327.2**

(22) Anmeldetag: **24.04.89**

(51) Int. Cl.4: **C07D 471/04 , A01N 43/90 , //(C07D471/04,241:00,221:00)**

(30) Priorität: **07.05.88 DE 3815617**

(43) Veröffentlichungstag der Anmeldung: **15.11.89 Patentblatt 89/46**

(84) Benannte Vertragsstaaten: **BE CH DE ES FR GB IT LI NL**

(71) Anmelder: **BAYER AG**

**D-5090 Leverkusen 1 Bayerwerk(DE)**

(72) Erfinder: **Heinemann, Ulrich, Dr.**
**Feldstrasse 18**
**D-5653 Leichlingen 1(DE)**
Erfinder: **Brandes, Wilhelm,Dr.**
**Eichendorffstrasse 3**
**D-5653 Leichlingen(DE)**
Erfinder: **Dutzmann, Stefan, Dr.**
**Leinenweberweg 33**
**D-4000 Düsseldorf 13(DE)**
Erfinder: **Hänssler, Gerd, Dr.**
**Am Arenzberg 58a**
**D-5090 Leverkusen 3(DE)**

(54) **4-Aza-1, 10-phenanthrolin-Derivate.**

(57) Neue 4-Aza-1,10-phenanthrolin-Derivate der Formel (I)

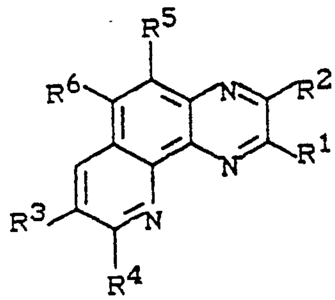

(I)

in welcher
$R^1$ bis $R^6$ die in der Beschreibung gegebenen Bedeutungen haben, deren Säureadditionssalze und Metallsalz-Komplexe und ihre Verwendung zur Bekämpfung von Schädlingen.

Die neuen 4-Aza-1,10-phenanthrolin-Derivate sind durch die Formel (I) allgemein definiert. Man kann sie nach Analogieverfahren herstellung, z. B. indem man geeignete 5-Aminochinoxaline mit geeigneten Aldehyden umsetzt oder indem man geeignete 7,8-Diaminochinoline mit geeigneten 1,2-Diketonen umsetzt.

EP 0 341 476 A2

# 4-Aza-1,10-phenanthrolin-Derivate

Die vorliegende Erfindung betrifft neue 4-Aza-1,10-phenanthrolin-Derivate, mehrere Verfahren zu ihrer Herstellung sowie ihre Verwendung in Schädlingsbekämpfungsmitteln, vor allem als Fungizide.

Bekannt sind die Synthese des unsubstituierten 4-Aza-1,10-phenanthrolins und seine Eigenschaft mit z. B. Fe (II) und gegebenenfalls Cu (I) Chelate zu bilden (vergl. J. A. C. S., 6297 - 6301, Vol. 81, 1959).

Es wurden nun neue 4-Aza-1,10-phenanthrolin-Derivate der Formel (I)

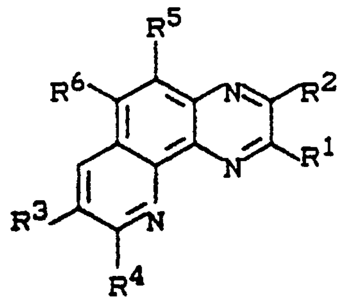

(I)

gefunden,
in welcher
$R^1$ und $R^2$ gleich oder verschieden sind und für Wasserstoff, Alkyl, gegebenenfalls substituiertes Aryl, gegebenenfalls substituiertes Hetaryl oder gemeinsam mit den Kohlenstoffatomen, an denen sie stehen, für eine Benzogruppierung stehen,
$R^3$ und $R^4$ gleich oder verschieden sind und für Wasserstoff oder Alkyl stehen und
$R^5$ und $R^6$ einzeln für Wasserstoff oder gemeinsam mit den Kohlenstoffatomen, an denen sie stehen, für eine Benzogruppierung stehen,
ausgenommen die Verbindung 4-Aza-1,10-phenanthrolin (vgl. J. A. C. S. 81, 6297 - 6301, (1959)),
sowie deren Säureadditions-Salze und Metallsalz-Komplexe.

Weiterhin wurde gefunden, daß man die neuen 4-Aza-1,10-phenanthrolin-Derivate der Formel (I)

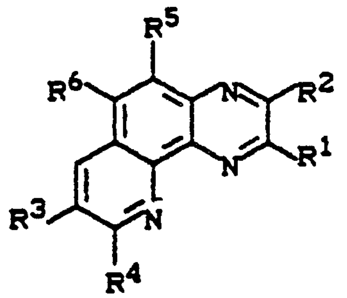

(I)

in welcher
$R^1$ und $R^2$ gleich oder verschieden sind und für Wasserstoff, Alkyl, gegebenenfalls substituiertes Aryl, gegebenenfalls substituiertes Hetaryl oder gemeinsam mit den Kohlenstoffatomen, an denen sie stehen, für eine Benzogruppierung stehen,
$R^3$ und $R^4$ gleich oder verschieden sind und für Wasserstoff oder Alkyl stehen und
$R^5$ und $R^6$ einzeln für Wasserstoff oder gemeinsam mit dem Kohlenstoffatomen, an denen sie stehen, für eine Benzogruppierung stehen, ausgenommen das 4-Aza-1,10-phenanthrolin,
sowie deren Säureadditions-Salze und Metallsalz-Komplexe erhält, wenn man
(A) 5-Aminochinoxaline der Formel (II)

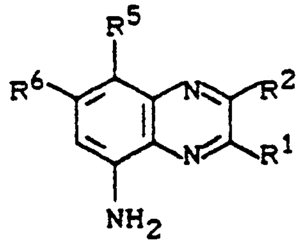

(II)

in welcher
$R^1$, $R^2$, $R^5$ und $R^6$ die oben angegebene Bedeutung haben,
wobei jedoch $R^1$, $R^2$, $R^5$ und $R^6$ nicht gleichzeitig für Wasserstoff stehen,

α) mit Aldehyden der Formel (III)

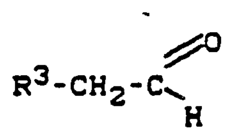

(III)

in welcher
$R^3$ die oben angegebene Bedeutung hat,
gegebenenfalls in Gegenwart von Verdünnungsmitteln sowie gegebenenfalls in Gegenwart von starken Säuren, insbesondere Lewis-Säuren, cyclisiert und die so erhaltenen Produkte gegebenenfalls in Säureadditions-Salze oder Metallsalz-Komplexe überführt, oder

ß) mit α,ß-ungesättigten Aldehyden der Formel (IIIa)

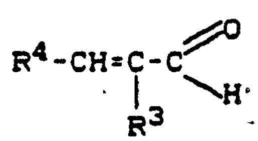

(IIIa)

in welcher
$R^3$ und $R^4$ die oben angegebene Bedeutung haben,
gegebenenfalls in Gegenwart von Verdünnungsmitteln und gegebenenfalls in Gegenwart einer starken Säure oder gegebenenfalls in Gegenwart eines Oxidationsmittels oder unter Wasserabscheidung gegebenenfalls in Gegenwart eines Katalysators cyclisiert und die so erhaltenen Produkte gegebenenfalls in Säureadditions-Salze oder Metallsalz-Komplexe überführt,
oder man erhält die 4-Aza-1,10-phenanthrolin-Derivate der formel (I), wenn man
(B) 7,8-Diaminochinoline der Formel (IV)

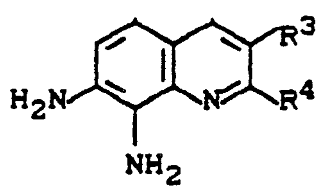

(IV)

in welcher
$R^3$ und $R^4$ die oben angegebene Bedeutung haben,
mit 1,2-Diketonen der Formel (V)

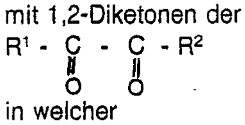

(V)

in welcher
$R^1$ und $R^2$ die oben angegebene Bedeutung haben,
gegebenenfalls in Gegenwart eines Verdünnungsmittels umsetzt und die so erhaltenen Produkte gegebenenfalls in Säureadditions-Salze oder Metallsalz-Komplexe überführt.

Außerdem wurde gefunden, daß sich die neuen 4-Aza-1,10-phenanthrolin-Derivate der Formel (I) und deren Säureadditions-Salze und Metallsalz-Komplexe durch starke biologische Eigenschaften auszeichnen.

Überraschenderweise zeigen die erfindungsgemäßen 4-Aza-1,10-phenanthrolin-Derivate der Formel (I) sowie deren Säureadditions-Salze und Metallsalz-Komplexe eine gute Wirkung gegen Schädlinge, insbesondere Pilze.

Die erfindungsgemäßen 4-Aza-1,10-phenanthrolin-Derivate stellen somit eine wertvolle Bereicherung der Technik dar.

Die erfindungsgemäßen 4-Aza-1,10-phenanthrolin-Derivate sind durch die Formel (I) allgemein definiert. Bevorzugt werden diejenigen Verbindungen der Formel (I),

in welcher

$R^1$ und $R^2$ gleich oder verschieden sind und für Wasserstoff, geradkettiges oder verzweigtes Alkyl mit 1 bis 4 Kohlenstoffatomen; gegebenenfalls einfach bis dreifach, gleich oder verschieden durch Halogen, Alkyl mit 1 bis 4 Kohlenstoffatomen oder Alkoxy mit 1 bis 4 Kohlenstoffato men substituiertes Phenyl; für einen gegebenenfalls einfach bis dreifach, gleich oder verschieden durch Halogen oder Alkyl mit 1 bis 4 Kohlenstoffatomen substituierten 5- oder 6-Ring Hetaryl mit 1 oder 2 gleichen oder verschiedenen Heteroatomen wie Sauerstoff, Schwefel und Stickstoff stehen, z. B. für Pyridyl, Pyrimidinyl oder Furanyl, oder gemeinsam mit den Kohlenstoffatomen, an denen sie stehen, für eine Benzogruppierung stehen,

$R^3$ und $R^4$ gleich oder verschieden sind und für Wasserstoff oder geradkettiges oder verzweigtes Alkyl mit 1 bis 4 Kohlenstoffatomen stehen und

$R^5$ und $R^6$ einzeln für Wasserstoff oder gemeinsam mit den Kohlenstoffatomen, an denen sie stehen, für eine Benzogruppierung stehen,

ausgenommen die Verbindung 4-Aza-1,10-phenanthrolin.

Besonders bevorzugt werden diejenigen Verbindungen der Formel (I), in welchen

$R^1$ und $R^2$ gleich oder verschieden sind und für Wasserstoff, Methyl, Ethyl, n- oder i-Propyl, n-, i-, s- oder t-Butyl; gegebenenfalls einfach oder zweifach, gleich oder verschieden durch Fluor, Chlor, Brom, Methyl, Ethyl, n- oder i-Propyl, Methoxy und/oder Ethoxy substituiertes Phenyl; für gegebenenfalls einfach bis drei fach, gleich oder verschieden durch Fluor, Chlor, Brom, Methyl und/oder Ethyl substituiertes Pyridyl, Pyrimidinyl oder Furanyl oder gemeinsam mit den Kohlenstoffatomen, an denen sie stehen, für eine Benzogruppierung stehen,

$R^3$ und $R^4$ gleich oder verschieden sind und für Wasserstoff, Methyl, Ethyl, n- oder i-Propyl, n-, i-, s- oder t-Butyl stehen und

$R^5$ und $R^6$ einzeln für Wasserstoff oder gemeinsam mit den Kohlenstoffatomen, an denen sie stehen, für eine Benzogruppierung stehen.

Bevorzugte erfindungsgemäße Verbindungen sind auch Additionsprodukte aus Säuren und denjenigen 4-Aza-1,10-phenanthrolin-Derivaten der Formel (I) in denen $R^1$, $R^2$, $R^3$, $R^4$, $R^5$ und $R^6$ diejenigen Bedeutungen haben, die im Zusammenhang mit der Beschreibung der erfindungsgemäßen Stoffe bereits vorzugsweise für diese Substituenten genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Schwefelsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salicylsäure, Sorbinsäure und Milchsäure, Ölsäure, Stearinsäure, gegebenenfalls einfach bis mehrfach durch Nitro oder Halogen substituierte Benzoesäure, Gluconsäure, Ascorbinsäure, Äpfelsäure, Sulfamidsäure, Sulfonsäuren, wie z. B. p-Toluolsulfonsäure, 1,5-Naphthalindisulfonsäure und Methansulfonsäure, sowie Imide, wie z. B. Phthalimid, Saccharin und Thiosaccharin.

Außerdem bevorzugte erfindungsgemäße Verbindungen sind auch Additionsprodukte aus Salzen von Metallen der I., II. und III. Hauptgruppe sowie des Zinns, sowie ferner Salze von Metallen der I., II., VII. und VIII. Nebengruppe des Periodensystems der Elemente und denjenigen 4-Aza-1,10-phenanthrolin-Derivaten der Formel (I), in denen $R^1$, $R^2$, $R^3$, $R^4$, $R^5$ und $R^6$ die Bedeutungen haben, die bereits im Zusammenhang mit der Beschreibung der erfindingsgemäßen Stoffe der Formel (I) vorzugsweise für diese Substituenten genannt wurden.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z. B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden 4-Aza-1,10-phenanthrolin-Derivate der allgemeinen Formel (I) genannt:

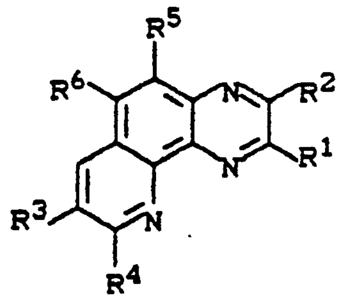

(I)

**Tabelle 1**

| $R^1$ | $R^2$ | $R^3$ | $R^4$ | $R^5$ | $R^6$ |
|---|---|---|---|---|---|
| $CH_3$ | $CH_3$ | H | $CH_3$ | H | H |
| $C_2H_5$ | $C_2H_5$ | H | $CH_3$ | H | H |
| H | H | $CH_3$ | $C_2H_5$ | H | H |
| H | H | $CH_3$ | $C_2H_5$ | -CH=CH-CH=CH- | |
| -CH=CH-CH=CH- | | $CH_3$ | $C_2H_5$ | H | H |
| 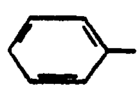 | H | H | $CH_3$ | -CH=CH-CH=CH- | |
| 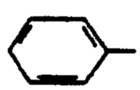 | H | H | $CH_3$ | H | H |
| $C_2H_5$ | $CH_3$ | H | $CH_3$ | H | H |

Tabelle 1 - Fortsetzung

| $R^1$ | $R^2$ | $R^3$ | $R^4$ | $R^5$ | $R^6$ |
|---|---|---|---|---|---|
| $n-C_3H_7-$ | $n-C_3H_7-$ | H | $CH_3$ | H | H |
| $n-C_3H_7-$ | $CH_3$ | $CH_3$ | $C_2H_5$ | H | H |
| $-CH=CH-CH=CH-$ | | H | $CH_3$ | H | H |
| $-CH=CH-CH=CH-$ | | H | $CH_3$ | $-CH=CH-CH=CH-$ | |
| 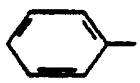 | 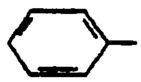 | $CH_3$ | $C_2H_5$ | H | H |
| 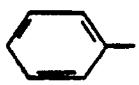 | 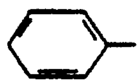 | H | $CH_3$ | H | H |
| H | H | $C_2H_5$ | $n-C_3H_7-$ | H | H |
| H | H | $i-C_3H_7-$ | $s-C_4H_9$ | H | H |
| $CH_3$ | $CH_3$ | $C_2H_5$ | $n-C_3H_7$ | H | H |
| $C_2H_5$ | $C_2H_5$ | $CH_3$ | $C_2H_5$ | H | H |
| $-CH=CH-CH=CH-$ | | $C_2H_5$ | $n-C_3H_7$ | H | H |
| $CH_3O$ 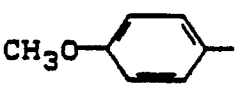 | $CH_3O$ 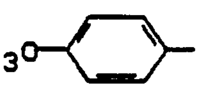 | $CH_3$ | $C_2H_5$ | H | H |
| Cl 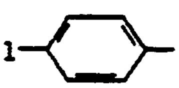 | Cl 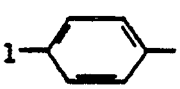 | $CH_3$ | $C_2H_5$ | H | H |
| 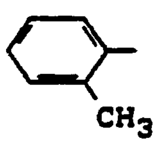 | 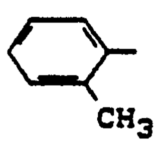 | H | $CH_3$ | H | H |
| $CH_3$ 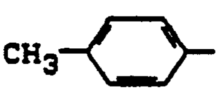 | $CH_3$ 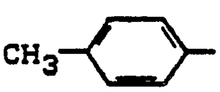 | H | $CH_3$ | H | H |

Tabelle 1 - Fortsetzung

| $R^1$ | $R^2$ | $R^3$ | $R^4$ | $R^5$ | $R^6$ |
|---|---|---|---|---|---|
| 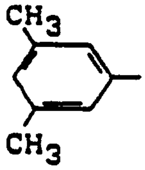 | 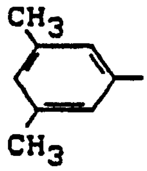 | $CH_3$ | $C_2H_5$ | H | H |
| 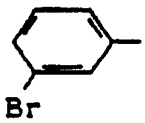 | 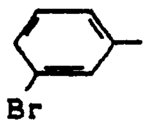 | $CH_3$ | $C_2H_5$ | H | H |
| 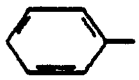 | $CH_3$ | $CH_3$ | $C_2H_5$ | H | H |
| 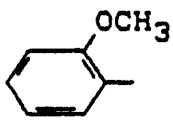 | 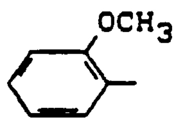 | H | $CH_3$ | -CH=CH-CH=CH | |
| 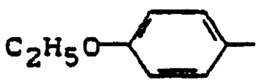 | H | H | $CH_3$ | -CH=CH-CH=CH | |
| 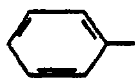 | $CH_3$ | $C_2H_5$ | $n-C_3H_7-$ | H | H |
| 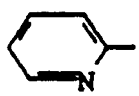 | 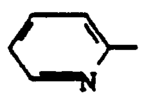 | $CH_3$ | $C_2H_5$ | H | H |
| 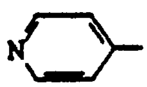 | 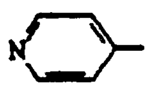 | $CH_3$ | $C_2H_5$ | H | H |
| 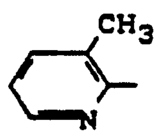 | 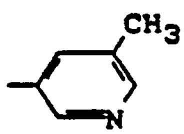 | H | $CH_3$ | H | H |

**Tabelle 1** - Fortsetzung

| $R^1$ | $R^2$ | $R^3$ | $R^4$ | $R^5$ | $R^6$ |
|---|---|---|---|---|---|
| 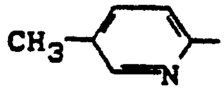 | 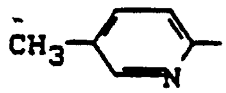 | $CH_3$ | $C_2H_5$ | H | H |
| 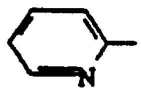 | 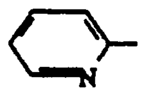 | H | $CH_3$ | -CH=CH-CH=CH | |
| 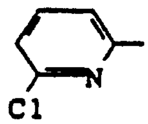 | 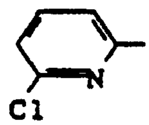 | $CH_3$ | $C_2H_5$ | H | H |
| 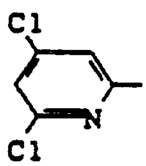 | 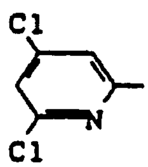 | $CH_3$ | $C_2H_5$ | H | H |
| 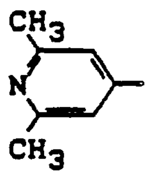 | 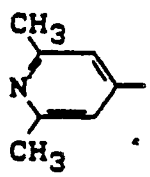 | $CH_3$ | $C_2H_5$ | H | H |
| 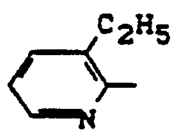 | 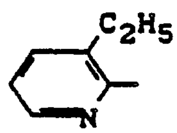 | H | $CH_3$ | H | H |
| 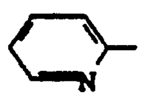 | H | $CH_3$ | $C_2H_5$ | H | H |
| 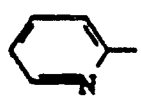 | H | H | $CH_3$ | H | H |
| 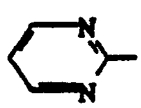 | 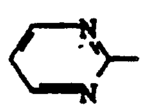 | $CH_3$ | $C_2H_5$ | H | H |

<u>Tabelle 1</u> - Fortsetzung

| $R^1$ | $R^2$ | $R^3$ | $R^4$ | $R^5$ | $R^6$ |
|---|---|---|---|---|---|
| 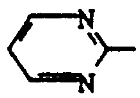 | 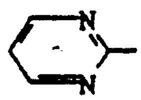 | H | $CH_3$ | H | H |
| 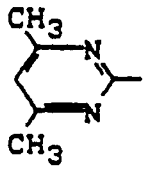 | 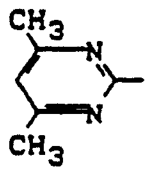 | H | $CH_3$ | -CH=CH-CH=CH | |
| 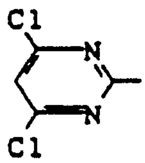 | 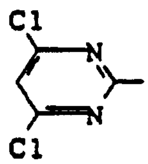 | $CH_3$ | $C_2H_5$ | H | H |
| 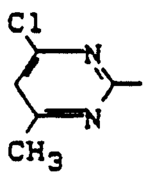 | 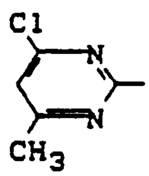 | $CH_3$ | $C_2H_5$ | H | H |
| 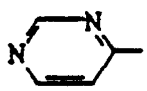 | 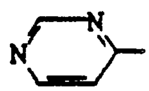 | $CH_3$ | $C_2H_5$ | H | H |
| 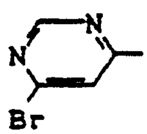 | 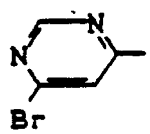 | H | $CH_3$ | H | H |
| 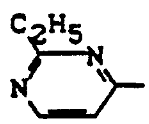 | 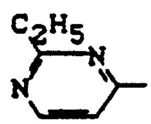 | $CH_3$ | $C_2H_5$ | H | H |
| 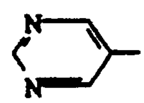 | 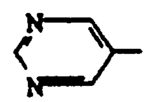 | $C_2H_5$ | n-$C_3H_7$ | H | H |

Tabelle 1 - Fortsetzung

| $R^1$ | $R^2$ | $R^3$ | $R^4$ | $R^5$ | $R^6$ |
|---|---|---|---|---|---|
| 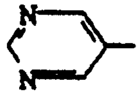 | H | $CH_3$ | $C_2H_5$ | H | H |
| 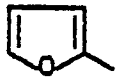 | 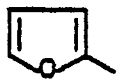 | $CH_3$ | $C_2H_5$ | H | H |
| 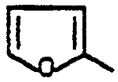 | 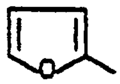 | H | $CH_3$ | H | H |
| 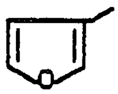 | 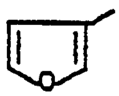 | $CH_3$ | $C_2H_5$ | H | H |
| 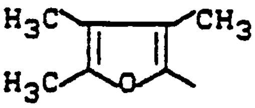 | 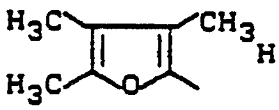 | H | $CH_3$ | H | H |
| 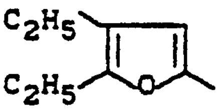 | 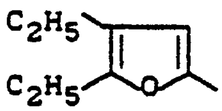 | $CH_3$ | $C_2H_5$ | H | H |
| 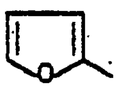 | 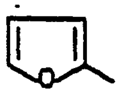 | H | $CH_3$ | -CH=CH-CH=CH | |
| 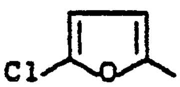 | 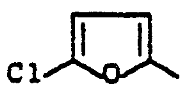 | $CH_3$ | $C_2H_5$ | H | H |
| 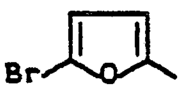 | 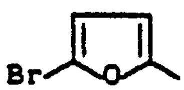 | $CH_3$ | $C_2H_5$ | H | H |
| 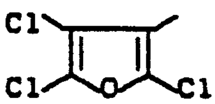 | 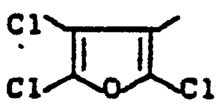 | H | $CH_3$ | H | H |

Tabelle 1 - Fortsetzung

| $R^1$ | $R^2$ | $R^3$ | $R^4$ | $R^5$ | $R^6$ |
|---|---|---|---|---|---|
| 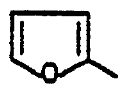 | H | $CH_3$ | $C_2H_5$ | H | H |
| 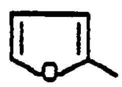 | H | H | $CH_3$ | H | H |
| 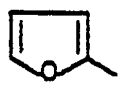 | H | H | $CH_3$ | -CH=CH-CH=CH | |
| 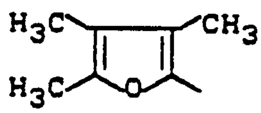 | H | $CH_3$ | $C_2H_5$ | H | H |
| 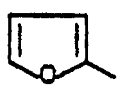 | $CH_3$ | $CH_3$ | $C_2H_5$ | H | H |
| 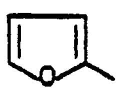 | $CH_3$ | H | $CH_3$ | H | H |
| 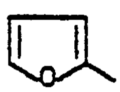 | $C_2H_5$ | H | $CH_3$ | H | H |

Verwendet man als Ausgangsstoffe beispielsweise 5-Amino-2,3-dimethyl-chinoxalin und Propionaldehyd, so läßt sich der Reaktionsablauf des Herstellungsverfahrens (A/α) durch das folgende Formelschema darstellen:

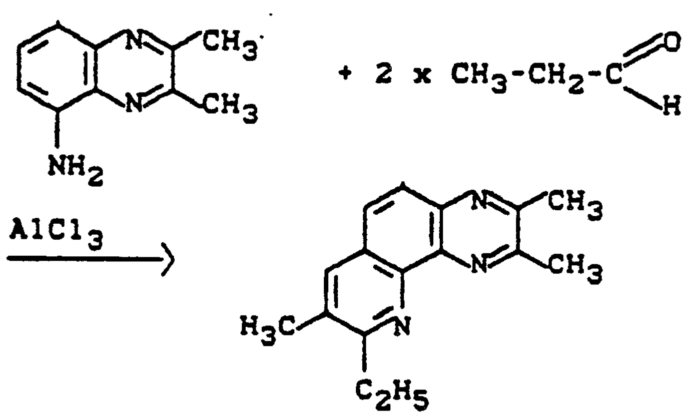


Verwendet man als Ausgangsstoffe beispielsweise 5-Amino-2,3-dimethyl-chinoxalin und Crotonaldehyd, so läßt sich der Reaktionsablauf des herstellungsverfahrens (A/ß) durch das folgende Formelschema darstellen:

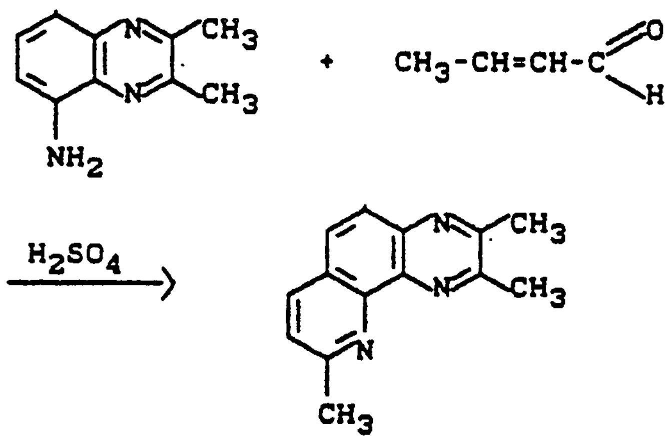


Verwendet man als Ausgangsstoffe beispielsweise 7,8-Diaminochinolin und Diacetyl, so läßt sich der Reaktions ablauf des Herstellungsverfahrens (B) durch das folgende Formelschema darstellen:

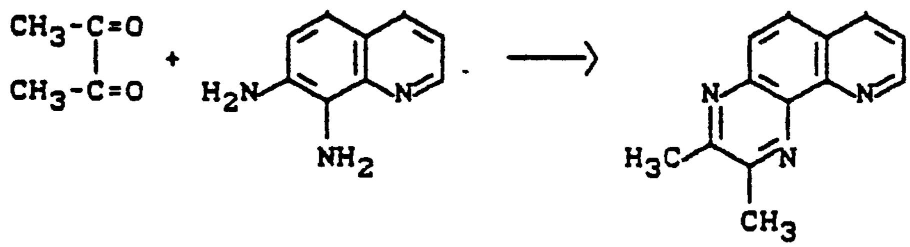

Die zur Durchführung der erfindungsgemäßen Verfahrensvarianten (A/α) und (A/ß) als Ausgangsstoffe benötigten 5-Aminochinoxaline sind durch die Formel (II) allgemein definiert. In dieser Formel (II) stehen $R^1$, $R^2$, $R^5$ und $R^6$ vorzugsweise bzw. insbesondere für diejenigen Substituenten, die oben bei der Beschreibung der neuen 4-Aza-1,10-Phenanthrolin-Derivate der Formel (I) als bevorzugt bzw. besonders bevorzugt für diese Reste genannt wurden.

Die 5-Aminochinoxaline der Formel (II) sind teilweise bekannt und/oder können nach bekannten Verfahren in einfacher, analoger Weise hergestellt werden (vgl. z. B. J. A. C. S 79, 2245 - 2248, (1957); J. Org. Chem. 31, 3384 - 3390, (1966) und Khim Geterotsikl. Soedin. 306 -310, (1976)), indem man beispielsweise die ebenfalls teilweise bekannten 5-Nitrochinoxaline der Formel (VI)

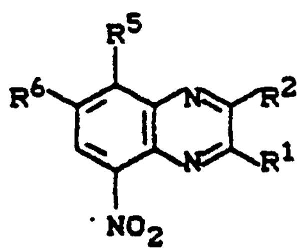
(VI)

in welcher
$R^1$, $R^2$, $R^5$ und $R^6$ die oben angegebene Bedeutung haben,
gegebenenfalls in Gegenwart eines Verdünnungsmittels in üblicher Art und Weise und in Gegenwart eines Katalysators, wie beispielsweise Raney-Nickel oder Zinn-II-Salzen, bei Temperaturen zwischen 20 °C und 150 °C und gegebenenfalls unter Druck zwischen 2 und 100 bar reduziert.

Die 5-Nitrochinoxaline der Formel (VI) sind teilweise bekannt und/oder können nach bekannten Verfahren in einfacher, analoger Weise hergestellt werden (vgl. z. B. J. Chem. Soc. 1953, 2822 - 2830), indem man beispielsweise
(C) o-Phenylendiamine der Formel (VII)

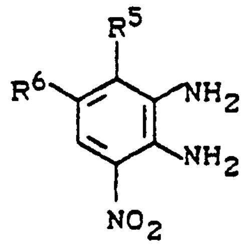

(VII)

in welcher
$R^5$ und $R^6$ die oben angegebene Bedeutung haben,
mit 1,2-Diketonen der Formel (V)

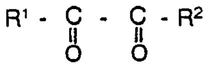

(V)

in welcher
$R^1$ und $R^2$ die oben angegebene Bedeutung haben,
gegebenenfalls in Gegenwart eines Verdünnungsmittels, wie beispielsweise Wasser, Essigsäure, Methanol oder Ethanol oder Gemischen aus diesen Lösungsmitteln, bei Temperaturen zwischen 50 °C und 150 °C umsetzt,
oder man erhält die 5-Nitrochinoxaline der Formel (VI), indem man
(D) o-Benzochinone der Formel (VIII)

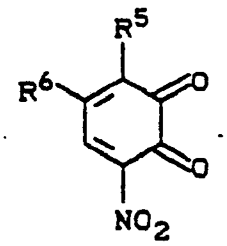

(VIII)

in welcher
$R^5$ und $R^6$ die oben angegebene Bedeutung haben
mit Ethylendiamin-Derivaten der Formel (IX)

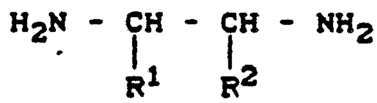

(IX)

in welcher
$R^1$ und $R^2$ die oben angegebene Bedeutung haben,
gegebenenfalls in Gegenwart eines Verdünnungsmittels wie beispielsweise Wasser, Eisessig, Methanol oder Ethanol oder Gemischen aus diesen Lösungsmitteln bei Temperaturen zwischen 40 °C und 140 °C umsetzt.

Die als Ausgangsstoffe gemäß Verfahren (C) und (D) benötigten Verbindungen der Formeln (V), (VII), (VIII) und (IX) sind allgemein bekannte Verbindungen der organischen Chemie.

Die außerdem als Ausgangsstoffe zur Herstellung der erfindungsgemäßen Verbindungen der Formel (I) gemäß Verfahren (A/α) und (A/ß) zu verwendenden Aldehyde sind durch die Formeln (III) und (IIIa) allgemein definiert. In den Formeln (III) und (IIIa) haben die Reste $R^3$ und $R^4$ vorzugsweise bzw. insbesondere diejenige Bedeutung, die oben im Zusammenhang mit der Beschreibung der erfindungsgemäßen Stoffe der Formel (I) als bevorzugt angegeben ist.

Die verbindungen der Formel (III) und (IIIa) sind allgemein bekannte Verbindungen der organischen Chemie.

Als Verdünnungsmittel zur Durchführung der Herstellungsverfahren (A/α) und (A/ß) kommen Mischungen von starken anorganischen oder organischen Säuren mit Wasser oder inerten organischen Lösungsmitteln in Frage. Säuren, welche zur Durchführung des Herstellungsverfahrens (A/α) oder (A/ß) verwendet werden können sind beispielsweise Schwefelsäure, Salzsäure, p-Toluolsulfonsäure und Lewis-Säuren, wie

beispielsweise Aluminiumchlorid und Eisen(III)chlorid, insbesondere Aluminiumchlorid.

Zur Durchführung des Herstellungsverfahrens (A/α) setzt man 1 Mol 5-Aminochinoxalin der Formel (II) mit annähernd äquimolaren Mengen einer Lewis-Säure um. Ein Überschuß der einen oder anderen Reaktionskomponente bringt keine wesentlichen Vorteile.

Zur Durchführung des Herstellungsverfahrens (A/α) oder (A/ß) können praktisch alle inerten organischen Lösungsmittel verwendet werden, insbesondere aliphatische und aromatische, gegebenenfalls halogenierte Kohlenwasserstoffe, wie beispielsweise, Benzin, Benzol, Toluol, Xylol, Chlorbenzol, Dichlorbenzol, Petrolether, Pentan, Hexan, Heptan, Ligroin oder Cyclohexan. Besonders bevorzugt sind Methylenchlorid und Chloroform.

Die Reaktionstemperaturen können bei der Durchführung der Herstellungsverfahren (A/α) und (A/ß) in einem größeren Bereich variiert werden. Im allgemeinen arbeitet man bei Temperaturen zwischen 20 °C und 180 °C, vorzugsweise bei Temperaturen zwischen 50 °C und 130 °C.

Als Oxidationsmittel zur Durchführung des Herstellungsverfahrens (A/ß) kommen die für derartige Reaktionen üblichen Oxidationsmittel in Frage; vorzugsweise verwendet man Arsensäure oder Nitrogruppen enthaltende aromatische Säuren oder deren Alkalimetall-Salze, wie beispielsweise 3-Nitrobenzolsulfonsäure.

Zur Durchführung der Herstellungsverfahren (A/α) und (A/ß) setzt man pro Mol 5-Aminochinoxalin der Formel (II) 1,0 bis 4,0 Mol, vorzugsweise 1,5 bis 3,5 Mol an Aldehyden der Formel (III) oder (IIIa) und gegebenenfalls 1,0 bis 2,0 Mol, vorzugsweise 1,0 Mol Oxidationsmittel ein. Die Reaktionsführung, Aufarbeitung und Isolierung der Reaktionsprodukte der Formel(I) erfolgt in allgemein üblicher Art und Weise.

Die außerdem als Ausgangsstoffe zur Herstellung der erfindungsgemäßen Verbindungen der Formel (I) gemäß Verfahren (B) zu verwendeten 7,8-Diaminochinoline sind durch die Formel (IV) allgemein definiert. In dieser Formel (IV) haben die Reste $R^3$ und $R^4$ vorzugsweise bzw. insbesondere diejenige Bedeutung, die oben im Zusammenhang mit der Beschreibung der erfindungsgemäßen Stoffe der Formel (I) als bevorzugt angegeben ist.

Die Verbindungen der Formel (IV) sind allgemein bekannte Verbindungen der organischen Chemie.

Die weiterhin als Ausgangsstoff zur Herstellung der erfindungsgemäßen Verbindungen der Formel (I) gemäß Verfahren (B) zu verwendenden 1,2-Diketone sind durch die Formel (V) allgemein definiert. In dieser Formel (V) haben die Reste $R^1$ und $R^2$ vorzugsweise bzw. insbesondere diejenige Bedeutung, die oben im Zusammenhang mit der Beschreibung der erfindungsgemäßen Stoffe der Formel (I) als bevorzugt angegeben ist.

Die Verbindungen der Formel (V) sind allgemein bekannte Verbindungen der organischen Chemie.

Als Verdünnungsmittel zur Durchführung des Herstellungsverfahrens (B) kommen alle für derartige Reaktionen üblichen inerten Lösungsmittel in Frage. Hierzu gehören insbesondere Wasser, Eisessig, Alkohol, wie Methanol und Ethanol, Dimethylformamid oder Dimethylsulfoxid, Acetonitril, Ether sowie aliphatische und aromatische, gegebenenfalls halogenierte Kohlenwasserstoffe, wie beispielsweise, Benzin, Benzol, Toluol, Xylol, Chlorbenzol, Dichlorbenzol, Petrolether, Pentan, Hexan, Heptan, Ligroin oder Cyclohexan. Es können aber auch Gemische aus den genannten Verdünnungsmitteln verwendet werden.

Die Reaktionstemperaturen können bei der Durchführung des Herstellungsverfahrens (B) in einem größeren Bereich variiert werden. Im allgemeinen arbeitet man be Temperaturen zwischen 20 °C und 180 °C, vorzugsweise bei Temperaturen zwischen 60 °C und 140 °C.

Zur Durchführung des Herstellungsverfahrens (B) setzt man auf 1 Mol 7,8-Diaminochinolin der Formel (IV) 1,0 bis 3,0 Mol, vorzugsweise 1,0 bis 1,5 Mol an 1,2-Diketonen der Formel (V) ein. Die Aufarbeitung und Isolierung des Reaktionsproduktes der Formel (I) erfolgt nach allgemein üblichen Methoden, wie beispielsweise durch Kristallisation, Absaugen und Trocknen.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z. B. durch Lösen einer Verbindung der allgemeinen Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke biologische Wirkung auf und können zur Bekämpfung von un erwünschten Schädlingen praktisch eingesetzt werden. Die Wirkstoffe sind z. B. für den Gebrauch als Pflanzenschutzmittel einsetzbar, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg protektiv zur Bekämpfung von Erysiphe-Arten an Gerste. Pyrenophora teres-Arten man Gerste und Pyricularia-Arten an Reis eingesetzt werden.

Hervorzuheben ist, daß die erfindungsgemäßen Wirkstoffe nicht nur protektiv sondern auch mit besonders gutem Erfolgt kurativ zur Bekämpfung von Phytophthora-Arten an Tomaten eingesetzt werden können.

Außerdem besitzen die erfindungsgemäßen Wirkstoffe gegen Venturia-Arten bei Äpfeln und Septoria nodorum und Cochliobolus sativus an Getreide sowie in-vitro eine gute Wirkung.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon,

Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxy ethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die Herstellung und die Verwendung der erfindungsgemäßen Wirkstoffe geht aus den folgenden Beispielen hervor.

## Herstellungsbeispiele

### Beispiel 1

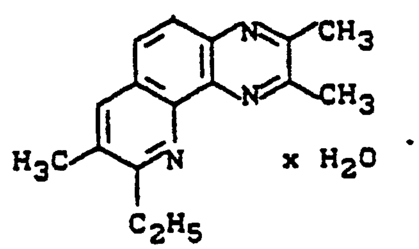

(I-1)

(Verfahren (A/α), 1. Stufe)

Zu einer Mischung von 13.3 g (0.076 Mol) 5-Amino-2,3-dimethyl-chinoxalin und 10,2 g (0,076 Mol) Aluminiumchlorid in 200 ml trockenem Methylenchlorid werden 9,3 g (0,160 Mol) Propionaldehyd, gelöst in 50 ml Methylenchlorid, bei Raumtemperatur zugetropft und anschließend 4 Stunden unter Rückfluß zum Sieden erhitzt. Nach dem Abkühlen wird das Lösungsmittel unter vermindertem Druck abdestilliert, der Rückstand in Wasser aufgenommen, 5 Minuten unter Rückfluß zum Sieden erhitzt, und dann heiß filtriert. Aus dem Filtrat scheiden sich beim Abkühlen Kristalle ab, die abgesaugt und getrocknet werden.

Man erhält 3,2 g (16 % der Theorie) 9-Ethyl-2,3,8-trimethyl-4-aza-1,10-phenanthrolin als Monohydrat vom Schmelzpunkt 137 °C bis 138 °C.

Beispiel 2

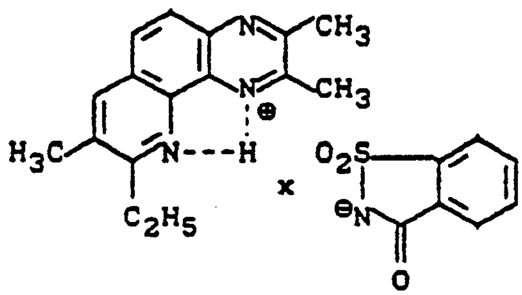

(I-2)

(Verfahren A/α, 2. Stufe, Salzbildung)

1,6 g (0,006 Mol) 9-Ethyl-2,3,8-trimethyl-4-aza-1,10-phenanthrolin (Beispiel 1) und 1,1 g (0,006 Mol) Saccharin werden in 50 ml trockenem Methylenchlorid 5 Minuten unter Rückfluß zum Sieden erhitzt. Nach dem Abkühlen wird filtriert, das Filtrat eingeengt, der Rückstand in Ether verrührt, abgesaugt und getrocknet.

Man erhält 2,3 g (85 % der Theorie) des Saccharin-Komplexsalzes des 9-Ethyl-2,3,8-trimethyl-4-aza-1,10-phenanthrolins mit dem Schmelzpunkt 198 °C bis 200 °C.

Beispiel 3

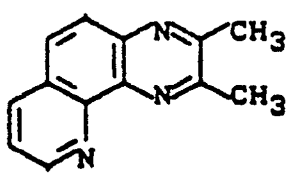

(I-3)

(Verfahren B)

4,4 g (0,027 Mol) 7,8-Diaminochinolin und 2,6 g (0,030 Mol) Diacetyl werden in 100 ml 10 %iger Essigsäure 1 Stunde unter Rückfluß zum Sieden erhitzt. Nach dem Abkühlen auf Raumtemperatur wird mit 500 ml Wasser verdünnt, mit konzentrierter Natronlauge alkalisch gestellt und die Reaktionsmischung über Nacht im Kühlschrank aufbewahrt. Die ausgefallenen Kristalle werden abgesaugt, mit wenig kaltem Wasser gewaschen und getrocknet.

Man erhält 2,1 g (37 % der Theorie) 2,3-Dimethyl-4-aza-1,10-phenanthrolin vom Schmelzpunkt 159 °C bis 162 °C.

In analoger Weise zu den in den Beispielen 1 bis 3 beschriebenen Methoden und unter Berücksichtigung der Angaben in den Beschreibungen zu den erfindungsgemäßen Verfahren, werden die nachfolgend in Tabelle 2 aufgeführten Endprodukte der Formel (I)

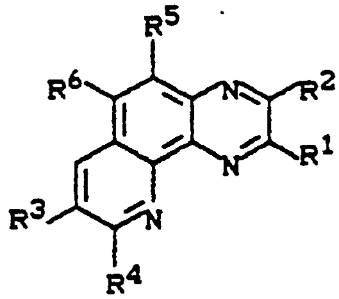

(I)

erhalten:

**Tabelle 2**

| Beisp.-Nr. | $R^1$ | $R^2$ | $R^3$ | $R^4$ | $R^5$ | $R^6$ | Metallkomplex Säureadditionssalz | Schmelzpunkt (°C) |
|---|---|---|---|---|---|---|---|---|
| I-4* | $C_2H_5/CH_3$ | $CH_3/C_2H_5$ | $CH_3$ | $C_2H_5$ | H | H | | 100 - 104 |
| I-5** | 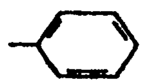 | H | $CH_3$ | $C_2H_5$ | H | H | | $^1$H-NMR δ = 7,15, 7,5; 1,55, 3,25; 2,6 |
| I-6 | -CH=CH-CH=CH- | | $CH_3$ | $C_2H_5$ | -CH=CH-CH=CH- | | | 172 - 175 |
| I-7* | $CH_3/C_2H_5$ | $C_2H_5/CH_3$ | $CH_3$ | $C_2H_5$ | H | H | $ZnCl_2$ | 238 - 250 |
| I-8 | $CH_3$ | $CH_3$ | H | $CH_3$ | H | H | | 185 - 188 |
| I-9 | $CH_3$ | $CH_3$ | $C_2H_5$ | H | H | H | | 149 - 153 |
| I-10 | $CH_3$ | $CH_3$ | H | $CH_3$ | H | H | | > 300 |
| I-11 | $CH_3$ | $CH_3$ | H | $CH_3$ | H | H | $CuCl_2$ | 208 - 210 |
| I-12 | $CH_3$ | $CH_3$ | H | $CH_3$ | H | H | Saccharat x 0,5 Ethanol | 204 - 207 |
| I-13 | $CH_3$ | $CH_3$ | H | $CH_3$ | H | H | Naphthalin-1,5-disulfonat | 280 (Zers.) |
| I-14 | $CH_3$ | $CH_3$ | $C_2H_5$ | H | H | H | $CuCl_2$ | 240 (Zers.) |
| I-15 | $CH_3$ | $CH_3$ | $C_2H_5$ | H | H | H | $ZnCl_2$ | > 300 |

**Tabelle 2** - Fortsetzung

| Beisp.- Nr. | $R^1$ | $R^2$ | $R^3$ | $R^4$ | $R^5$ | $R^6$ | Metallkomplex Säureadditionssalz | Schmelzpunkt (°C) |
|---|---|---|---|---|---|---|---|---|
| I-16 | $CH_3$ | $CH_3$ | $C_2H_5$ | H | H | H | Citrat | 142 - 147 |
| I-17 | $C_2H_5$ | $C_2H_5$ | H | $CH_3$ | H | H | | 155 - 157 |
| I-18 | $C_2H_5$ | $C_2H_5$ | $C_2H_5$ | H | H | H | | 85 - 87 |
| I-19 | 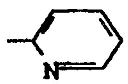 | 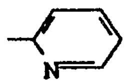 | H | $CH_3$ | H | H | 1/2 $H_2O$ | 195 - 197 |
| I-20 | $C_2H_5$ | $C_2H_5$ | H | $CH_3$ | H | H | $ZnCl_2$ | 273 - 279 |
| I-21 | $C_2H_5$ | $C_2H_5$ | $C_2H_5$ | H | H | H | $ZnCl_2$ | 305 - 308 |
| I-22 | $C_2H_5$ | $C_2H_5$ | H | $CH_3$ | H | H | Saccharat | 103 - 105 |
| I-23 | $C_2H_5$ | $C_2H_5$ | $CH_3$ | $C_2H_5$ | H | H | | 131 - 133 |
| I-24 | 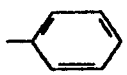 | 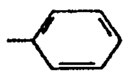 | H | $CH_3$ | H | H | $H_2O$ | 219 - 221 |
| I-25 | 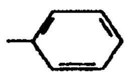 | 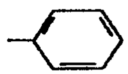 | H | $CH_3$ | H | H | $CuCl_2$ x 1/2 $H_2O$ | 200 (Zers.) |
| I-26 | 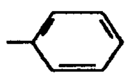 | H | H | $CH_3$ | H | H | | $^1$H-NMR $\delta$ = 2,95 (s, 3H) |

* Verbindung (I-4) und (I-7) liegen als Isomerengemische (Stellungsisomere) vor.

** Die $^1$H-NMR-Spektren wurden in Deuterochloroform ($CDCl_3$) mit Tetramethylsilan (TMS) als innerem Standard aufgenommen. Angegeben ist die chemische Verschiebung als $\delta$-Wert in ppm.

Herstellung der Ausgangsstoffe

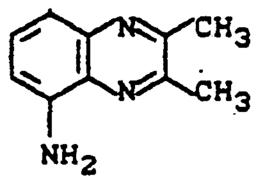

(II-1)

Zu 106,0 g (0,470 Mol) Zinn(II)chlorid-Dihydrat in 300 ml konzentrierter Salzsäure werden 27,8 g (0,136 Mol) 2,3-Dimethyl-5-nitro-chinoxalin portionsweise zugegeben. Die Temperatur steigt dabei auf 50 °C an, und das Reaktionsgemisch wird 1 Stunde bei 50 °C gehalten. Nach dem Abkühlen wird abgesaugt, der Filterkuchen in 100 ml konzentrierter Natronlauge suspendiert und nach Zugabe von 100 ml Wasser ca. 30 Minuten gerührt. Anschließend wird abgesaugt und der Filterkuchen in 150 ml Methylenchlorid gelöst. Die organische Phase wird mit Natriumsulfat getrocknet, das Lösungsmittel unter vermindertem Druck abdestilliert, der Rückstand in Petrolether aufgenommen, abgesaugt und getrocknet.

Man erhält 18,6 g (79 % der Theorie) 5-Amino-2,3-dimethyl-chinoxalin vom Schmelzpunkt 162 °C bis 163 °C.

In analoger Weise zu dem in Beispiel (II-1) beschriebenen Verfahren und unter Berücksichtigung der Angaben in den Beschreibungen zu den Verfahren werden die nachfolgend in Tabelle 3 aufgeführten Zwischenprodukte der Formel (II)

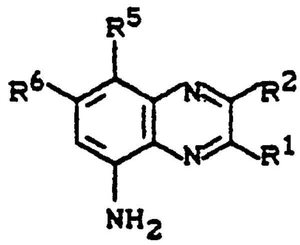

(II)

erhalten.

Tabelle 3

| Beisp.-Nr. | $R^1$ | $R^2$ | $R^5$ | $R^6$ | Schmelzpunkt (°C) |
|---|---|---|---|---|---|
| II-2* | $CH_3/C_2H_5$ | $C_2H_5/CH_3$ | H | H | 91 - 94 |
| II-3 | 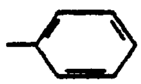 | H | H | H | 88 - 90 |
| II-4 | 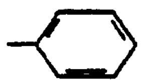 | 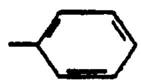 | H | H | 242 - 245 |
| II-5 | 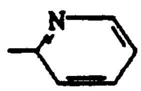 | 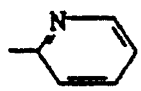 | H | H | 110 (Zers.) |
| II-6 | $C_2H_5$ | $C_2H_5$ | H | H | 68 - 70 |
| II-7 | H | 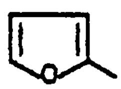 | H | H | 145 - 146 |
| II-8** | 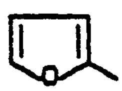 | 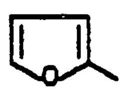 | H | H | $^1$H-NMR $\delta$ = 6,5 - 6,6 |

* Verbindung (II-2) liegt als Isomerengemisch vor.

** Die $^1$H-NMR-Spektren wurden in Deuterochloroform ($CDCl_3$) mit Tetramethylsilan (TMS) als innerem Standard aufgenommen. Angegeben ist die chemische Verschiebung als $\delta$-Wert in ppm.

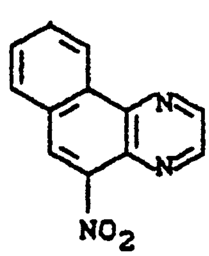

(VI-1)

20,3 g (0,10 Mol) 3-Nitro-o-naphthochinon und 6,0 g (0,10 Mol) Ethylendiamin werden in 150 ml

Eisessig 2 Stunden bei 100 °C gerührt. Nach dem Abkühlen auf Raumtemperatur wird das Reaktionsgemisch auf 500 ml Wasser gegossen, der ausgefallene Niederschlag abgesaugt, mit Wasser gewaschen und getrocknet. Der Rückstand wird chromatographisch über 100 g Aluminiumoxid (Laufmittel Methylenchlorid) gereinigt.

Man erhält 3,5 g (16 % der Theorie) 5-Nitro-1,4-phenanthrolin vom Schmelzpunkt 163 °C - 165 °C.

In analoger Weise zu dem in Beispiel (VI-1) beschriebenen Verfahren und unter Berücksichtigung der Angaben in den Beschreibungen zu den Verfahren, werden die nachfolgend in Tabelle 4 aufgeführten Zwischenprodukte der Formel (VI)

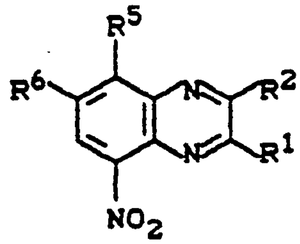

(VI)

erhalten:

Tabelle 4

| Beisp.-Nr. | $R^1$ | $R^2$ | $R^5$ | $R^6$ | Schmelzpunkt (°C) |
|---|---|---|---|---|---|
| VI-2 | $CH_3$ | $CH_3$ | H | H | 132 - 134 |
| VI-3* | $CH_3/C_2H_5$ | $C_2H_5/CH_3$ | H | H | 91 - 94 |
| VI-4 | 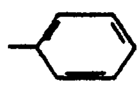 | H | H | H | 170 - 171 |
| VI-5 | 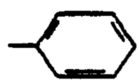 | 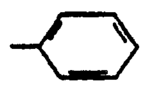 | H | H | 168 - 171 |
| VI-6 | 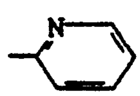 | 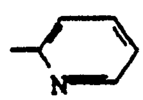 | H | H | 198 - 200 |
| VI-7 | $C_2H_5$ | $C_2H_5$ | H | H | 97 - 100 |
| VI-8 | 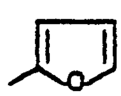 | 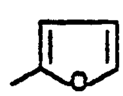 | H | H | 117 -120 |

* Die Verbindung (VI-3) liegt als Isomerengemisch vor.

Anwendungsbeispiele

Beispiel A

Pyrenophora teres-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächsaus bei einer Temperatur von ca. 20 °C und einer relativen Luftfeuchtigkeit von ca. 80 °C aufgestellt.

7 Tagen nach der Inokulation erfolgt die Auswertung.

In diesem Test zeigt z. B. der erfindungsgemäße Stoff (I-2) einen Wirkungsgrad von 100 % gegenüber der unbehandelten Kontrolle bei einer Wirkstoffkonzentration von 0,025 Gew.-% in der Spritzbrühe.

Beispiel B

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächsaus bei einer Temperatur von ca. 20 °C und einer relativen Luftfeuchtigkeit von ca. 80 °C aufgestellt, um die Entwicklung von Mehltaupusteln zu begünstigen.

7 Tage nach der Inokulation erfolgt die Auswertung.

In diesem Test zeigt z. B. der erfindungsgemäße Stoff (I-2) einen Wirkungsgrad von 88 % gegenüber der unbehandelten Kontrolle bei einer Wirkstoffkonzentration von 0,025 Gew.-% in der Spritzbrühe.

Beispiel C

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

In diesem Test zeigen z. B. der erfindungsgemäße Stoffe (I-1), (I-2), (I-3), (I-4), (I-5) und (I-7) einen Wirkungsgrad von 100 % gegenüber der unbehandelten Kontrolle bei einer Wirkstoffkonzentration von 0,025 %.

Beispiel D

Phytophthora-Test (Tomate)/kurativ

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit der wäßrigen Sporensuspension von Phytophthora infestans inokuliert. Die Pflanzen verbleiben 7 Stunden bei 20 ° C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einem Inkubationskabine mit 100% relativer Luftfeuchtigkeit und ca. 20 °C aufgestellt.

3 Tage nach der Inokulation erfolgt die Auswertung.

In diesem Test zeigt z. B. der erfindungsgemäße Verbindung (I-1) einen Wirkungsgrad von fast 90 % bei einer Wirkstoffkonzentration von 50 ppm.

## Ansprüche

1. 4-Aza-1,10-phenanthrolin-Derivate der Formel (I)

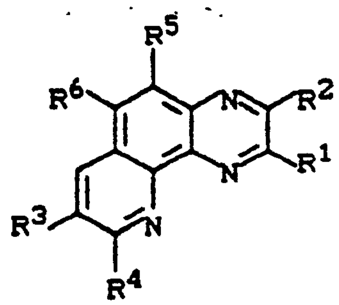

(I)

in welcher

$R^1$ und $R^2$ gleich oder verschieden sind und für Wasserstoff, Alkyl, gegebenenfalls substituiertes Aryl, gegebenenfalls substituiertes Hetaryl oder gemeinsam mit den Kohlenstoffatomen, an denen sie stehen, für eine Benzogruppierung stehen,

$R^3$ und $R^4$ gleich oder verschieden sind und für Wasserstoff oder Alkyl stehen und

$R^5$ und $R^6$ einzeln für Wasserstoff oder gemeinsam mit den Kohlenstoffatomen, an denen sie stehen, für, eine Benzogruppierung stehen,

ausgenommen die Verbindung 4-Aza-1,10-phenanthrolin

sowie deren Säureadditions-Salze und Metallsalz-Komplexe.

2. 4-Aza-1,10-phenanthrolin-Derivate gemäß Anspruch 1, wobei in der formel (I)

$R^1$ und $R^2$ gleich oder verschieden sind und für Wasserstoff, geradkettiges oder verzweigtes Alkyl mit 1 bis 4 Kohlenstoffatomen; gegebenenfalls einfach bis dreifach, gleich oder verschieden durch Halogen, Alkyl mit 1 bis 4 Kohlenstoffatomen oder Alkoxy mit 1 bis 4 Kohlenstoffatomen substituiertes Phenyl; für einen gegebenenfalls einfach bis dreifach, gleich oder verschieden durch Halogen oder Alkyl mit 1 bis 4 Kohlenstoffatomen substituierten 5- oder 6-Ring Hetaryl mit 1 oder 2 gleichen oder verschiedenen

Heteroatomen stehen oder gemeinsam mit den Kohlenstofatomen, an denen sie stehen, für eine Benzogruppierung stehen,
$R^3$ und $R^4$ gleich oder verschieden sind und für Wasserstoff oder geradkettiges oder verzweigtes Alkyl mit 1 bis 4 Kohlenstoffatomen stehen und
$R^5$ und $R^6$ einzeln für Wasserstoff oder gemeinsam mit den Kohlenstoffatomen, an denen sie stehen, für eine Benzogruppierung stehen,
ausgenommen die Verbindung 4-Aza-1,10-phenanthrolin.

3. 4-Aza-1,10-phenanthrolin-Derivate gemäß Anspruch 1, wobei in der Formel (I)
$R^1$ und $R^2$ gleich oder verschieden sind und für Wasserstoff, geradkettiges oder verzweigtes Alkyl mit 1 bis 4 Kohlenstoffatomen; gegebenenfalls einfach bis dreifach, gleich oder verschieden durch Halogen, Alkyl mit 1 bis 4 Kohlenstoffatomen oder Alkoxy mit 1 bis 4 Kohlenstoffatomen substituiertes Phenyl; für gegebenenfalls einfach bis dreifach, gleich oder verschieden durch Halogen oder Alkyl mit 1 bis 4 Kohlenstoffatomen substituiertes Pyridyl, Pyrimidinyl oder Furanyl oder gemeinsam mit den Kohlenstoffatomen, an denen sie stehen, für eine Benzogruppierung stehen,
$R^3$ und $R^4$ gleich oder verschieden sind und für Wasserstoff oder geradkettiges oder verzweigtes Alkyl mit 1 bis 4 Kohlenstoffatomen stehen und
$R^5$ und $R^6$ einzeln für Wasserstoff oder gemeinsam mit den Kohlenstoffatomen, an denen sie stehen, für eine Benzogruppierung stehen,
ausgenommen die Verbindung 4-Aza-1,10-phenanthrolin.

4. 4-Aza-1,10-phenanthrolin-Derivate gemäß Anspruch 1, wobei in der Formel (I)
$R^1$ und $R^2$ gleich oder verschieden sind und für Wasserstoff, Methyl, Ethyl, n- oder i-Propyl, n-, i-, s- oder t-Butyl; gegebenenfalls einfach oder zweifach, gleich oder verschieden durch Fluor, Chlor, Brom, Methyl, Ethyl, n- oder i-Propyl, Methoxy und/oder Ethoxy substituiertes Phenyl; für gegebenenfalls einfach bis dreifach, gleich oder verschieden durch Fluor, Chlor, Brom, Methyl und/oder Ethyl substituiertes Pyridyl, Pyrimidinyl oder Furanyl oder gemeinsam mit den Kohlenstoffatomen, an denen sie stehen, für eine Benzogruppierung stehen,
$R^3$ und $R^4$ gleich oder verschieden sind und für Wasserstoff, Methyl, Ethyl, n- oder i-Propyl, n-, i-, s- oder t-Butyl stehen und
$R^5$ und $R^6$ einzeln für Wasserstoff oder gemeinsam mit den Kohlenstoffatomen, an denen sie stehen, für eine Benzogruppierung stehen.

5. Verfahren zur Herstellung von 4-Aza-1,10-phenanthrolin-Derivaten der Formel (I)

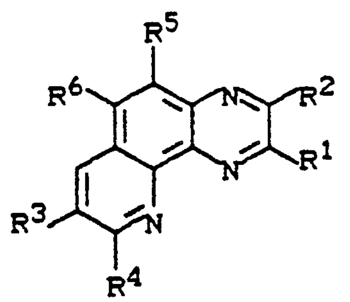

(I)

in welcher
$R^1$ und $R^2$ gleich oder verschieden sind und für Wasserstoff, Alkyl, gegebenenfalls substituiertes Aryl, gegebenenfalls substituiertes Hetaryl oder gemeinsam mit den Kohlenstoffatomen, an denen sie stehen, für eine Benzogruppierung stehen,
$R^3$ und $R^4$ gleich oder verschieden sind und für Wasserstoff oder Alkyl stehen und
$R^5$ und $R^6$ einzeln für Wasserstoff oder gemeinsam mit dem Kohlenstoffatomen, an denen sie stehen, für eine Benzogruppierung stehen,
sowie deren Säureadditions-Salze und Metallsalz-Komplexe, ausgenommen das 4-Aza-1,10-phenanthrolin, dadurch gekennzeichnet, daß man
(A) 5-Aminochinoxaline der Formel (II)

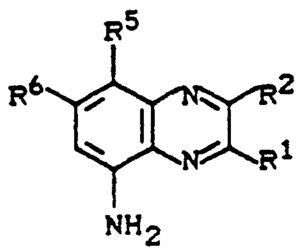

(II)

in welcher
$R^1$, $R^2$, $R^5$ und $R^6$ die oben angegebene Bedeutung haben
wobei jedoch $R^1$, $R^2$, $R^5$ und $R^6$ nicht gleichzeitig für Wasserstoff stehen,
α) mit Aldehyden der Formel (III)

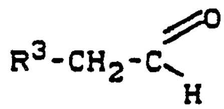

(III)

in welcher
$R^3$ die oben angegebene Bedeutung hat,
gegebenenfalls in Gegenwart von Verdünnungsmitteln sowie gegebenenfalls in Gegenwart von starken Säuren, cyclisiert und die so erhaltenen Produkte gegebenenfalls in Säureadditions-Salze oder Metallsalz-Komplex überführt, oder
ß) mit α,ß-ungesättigten Aldehyden der Formel (IIIa)

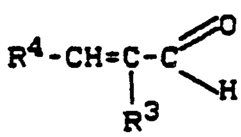

(IIIa)

in welcher
$R^3$ und $R^4$ die oben angegebene Bedeutung haben,
gegebenenfalls in Gegenwart von Verdünnungsmitteln und gegebenenfalls in Gegenwart einer starken Säure oder gegebenenfalls in Gegenwart eines Oxidationsmittels oder unter Wasserabscheidung gegebenenfalls in Gegenwart eines Katalysators cyclisiert und die so erhaltenen Produkte gegebenenfalls in Säureadditions-Salze oder Metallsalz-Komplexe überführt,
oder daß man
(B) 7,8-Diaminochinoline der Formel (IV)

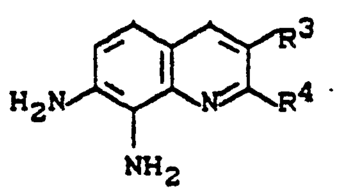

(IV)

in welcher
$R^3$ und $R^4$ die oben angegebene Bedeutung haben,
mit 1,2-Diketonen der Formel (V)

$$R^1 - \underset{\overset{\|}{O}}{C} - \underset{\overset{\|}{O}}{C} - R^2 \qquad (V)$$

in welcher
$R^1$ und $R^2$ die oben angegebene Bedeutung haben, gegebenenfalls in Gegenwart eines Verdünnungsmittels umsetzt und die so erhaltenen Produkte gegebenenfalls in Säureadditions-Salze oder Metallsalz-Komplexe

überführt.

6. Schädlingsbekämpfungsmittel, gekennzeichnet durch einen Gehalt an mindestens einem 4-Aza-1,10-phenanthrolin-Derivat der Formel (I) nach den Ansprüchen 1 und 5.

7. Verwendung von 4-Aza-1,10-phenanthrolin-Derivaten der Formel (I) nach den Ansprüchen 1 und 5 zur Bekämpfung von Schädlingen.

8. Verfahren zur Bekämpfung von Schädlingen, dadurch gekennzeichnet, daß man 4-Aza-1,10-phenanthrolin-Derivate der Formel (I) nach den Ansprüchen 1 und 5 auf Schädlinge und/oder ihren Lebensraum einwirken läßt.

9. Verfahren zur Herstellung von Schädlingsbekämpfungsmitteln, dadurch gekennzeichnet, daß man 4-Aza-1,10-phenanthrolin-Derivate der Formel (I) nach den Ansprüchen 1 und 5 mit Streckmitteln und/oder oberflächenaktiven Mitteln vermischt.